## Europäisches Patentamt

## European Patent Office

## Office européen des brevets

(11) Veröffentlichungsnummer: **0 045 407**
**A1**

(12) **EUROPÄISCHE PATENTANMELDUNG**

(21) Anmeldenummer: **81105512.8**

(22) Anmeldetag: **14.07.81**

(51) Int. Cl.³: **G 01 N 21/90**

(30) Priorität: **01.08.80 DE 3029678**

(43) Veröffentlichungstag der Anmeldung:
**10.02.82 Patentblatt 82/6**

(84) Benannte Vertragsstaaten:
**FR NL**

(71) Anmelder: **SIEMENS AKTIENGESELLSCHAFT Berlin und München**
**Postfach 22 02 61**
**D-8000 München 22(DE)**

(72) Erfinder: **Marguerre, Hans-Helmut, Dipl.-Phys.**
**Mähfeldstrasse 23**
**D-7541 Straubenhardt 4(DE)**

(54) Verfahren und Annnnordnung zur optisch-elektronischen Ermittlung von Verunreinigungen im Bodenbereich von transparenten Behältern.

(57) Bei der Bodenkontrolle von transparenten Behältern, insbesondere von Glasflaschen, auf Fremdkörper mittels einer Fernsehkamera mit nachgeschalteter Auswerteeinrichtung wird der Behälterboden (10) nicht nur parallel zur Behälterachse durchleuchtet, sondern auch unter einer Neigung von 10 bis 45° und gegebenenfalls 5 bis 15°. Die Leuchtdichte der geneigten Lichtstrahlen ist wesentlich größer als die Leuchtdichte der parallel zur Behälterachse verlaufenden Lichtstrahlen. Dadurch wird eine Aufhellung von Prägungen und Riffelungen am Behälterboden und des Übergangs Behälterboden/Behälterwand erzielt und die Fehlererkennung verbessert. Die Beleuchtung erfolgt mit Hilfe einer Blitzlampe (24), die von einem einseitig offenen Rohrkörper (20) umgeben ist. Die Innenwand des Rohrkörpers ist mit einem die Lichtstrahlen diffus streuenden Belag (21) versehen. Weiterhin sind der Blitzlampe Flächen (22) und/oder Blenden am Boden und/oder in einer Zwischenebene des Rohrkörpers (20) und/oder konzentrisch zur Achse des Rohrkörpers zugeordnet, die eine spiegelnde und/oder diffus streuende Oberfläche (22) aufweisen.

FIG 3
COMPLETE DOCUMENT

Croydon Printing Company Ltd.

SIEMENS AKTIENGESELLSCHAFT          Unser Zeichen
Berlin und München                  VPA 80 P 3534 E


Verfahren und Anordnung zur optisch-elektronischen
Ermittlung von Verunreinigungen im Bodenbereich
von transparenten Behältern

1. Anwendungsgebiet der Erfindung

Die Erfindung liegt auf dem Gebiet der optischen Meßtechnik und ist bei der optisch-elektronischen Erfassung
von Verunreinigungen im Bodenbereich von transparenten
Behältern anzuwenden. Ein spezielles Anwendungsgebiet ist
die Bodenkontrolle von Flaschen auf Fremdkörper.

2. Technischer Hintergrund

Zur Feststellung von Flecken auf kreisförmigen Objekten,
insbesondere zur Überprüfung der Reinheit von Flaschenböden, ist eine Anordnung bekannt, bei der die Flasche
auf einer transparenten Unterlage steht und bei der sich
unter dieser Unterlage eine blitzartige Beleuchtungseinrichtung befindet. Die von einer mit einem Reflektor
versehenen Blitzlampe ausgehenden Lichtstrahlen werden
mittels eines Spiegels umgelenkt und treffen parallel
oder nahezu parallel zur Achse der Flasche auf den Flaschenboden auf. Der so erhellte Flaschenboden wird mittels einer Fernsehkamera durch die Flaschenöffnung hindurch beobachtet und das Videosignal der Fernsehkamera
wird optisch (Monitor) oder elektronisch (Auswerteeinrichtung) ausgewertet (DE-OS 28 48 316). Mit dieser Anordnung lassen sich Fremdkörper und Flecken auf dem
Flaschenboden gut ermitteln, sofern der Flaschenboden
eben ist. Liegen jedoch Unebenheiten wie Prägungen oder
Riffelungen vor, so werden diese wegen der dort stattfindenden Lichtbrechung gegebenenfalls als Fremdkörper oder Fleck erkannt, was unerwünscht ist. Auch die Übergangszone Flaschenboden/Flaschenwand erscheint im Fernsehbild als dunkle Zone, so daß hier die Fehlererkennung ungenau ist. Die Fehlererkennung in dieser Zone ist aber von besonderer Bedeutung bei gewölbten Flaschenböden.

Zur Prüfung von Flaschenböden auf Verunreinigungen und Bruchstellen ist eine weitere Anordnung vorgeschlagen worden, die sich durch eine sehr flache, nur wenig Raum erfordernde Beleuchtungseinrichtung auszeichnet. Diese besteht aus mehreren gleichzeitig ansteuerbaren Lumineszenzdioden, die in konzentrischen Ringen in einer Fläche angeordnet sind. Zum Ausgleich unterschiedlicher Transmissionsgrade des Flaschenbodens in der Mitte und am Rand kann dabei der gegenseitige Abstand der Dioden in den äußeren Bereichen kleiner als in den mittleren Bereichen gewählt sein (DE-ANM P 29 09 061.7).

3. Darstellung der Erfindung

a) Technische Aufgabe

Der Erfindung liegt die Aufgabe zugrunde, bei der Ermittlung von Verunreinigungen im Bodenbereich von transparenten Behältern, insbesondere von Glasflaschen, sicherzustellen, daß Unebenheiten am Behälterboden nicht als Verunreinigung erkannt werden.

b) Lösung

Ausgehend von einem Verfahren, bei dem der Behälterboden von unten beleuchtet sowie durch die Öffnung des Behälters mittels einer Fernsehkamera beobachtet und das Videosignal der Fernsehkamera elektronisch ausgewertet wird und bei dem die Lichtstrahlen des zur Beleuchtung dienenden Lichtbündels parallel oder annähernd parallel zur senkrechten Achse des Behälters verlaufen, ist zur Lösung der genannten Aufgabe gemäß der Erfindung vorgesehen, daß der Behälterboden mit Hilfe wenigstens eines weiteren Lichtbündels beleuchtet wird, dessen Lichtstrahlen von der senkrechten Achse des Behälters rotationssymmetrisch um wenigstens 5 $^{\circ}$ abweichen und dessen Leuchtdichte um ein Vielfaches größer als die Leuchtdichte des erstgenannten Lichtbündels ist.

c) Vorteile und weitere Ausgestaltungen

Bei einem derart ausgebildeten Verfahren wird durch Verwendung wenigstens eines weiteren Lichtbündels eine Aufhellung derjenigen Stellen des Behälterbodens erreicht, die aufgrund ihrer geometrischen Struktur bei achsparalleler Lichteinstrahlung dunkler als die übrigen Bereiche erscheinen. Die erzielte Aufhellung resultiert aus einer Brechung der schräg einfallenden Lichtstrahlen an den Unebenheiten des Behälterbodens sowie einer Reflexion an der Behälterwand in Richtung auf das Objektiv der Fernsehkamera. Das Maß der Aufhellung hängt dabei sowohl vom Einstrahlwinkel des weiteren Lichtbündels als auch von dessen Leuchtdichte ab. So hat sich gezeigt, daß zur Aufhellung des Grenzbereiches Behälterboden/Behälterwand eine Abweichung des Einstrahlwinkels von der Behälterachse um ca. 5 bis 15 $^{\circ}$ sinnvoll ist, wobei die Abweichung der einzelnen Lichtstrahlen über diesen Bereich streuen kann. Hinsichtlich der Leuchtdichte ist es vorteilhaft, wenn diese ein Vielfaches, vorzugsweise das Zehnfache der Leuchtdichte des parallel zur Behälterachse verlaufenden Lichtbündels ist.

Zur Aufhellung von Prägungen und Riffelungen im Behälterboden ist dagegen die Verwendung eines weiteren Lichtbündels sinnvoll, dessen Lichtstrahlen von der senkrechten Achse des Behälters um ca. 10 bis 45 $^{\circ}$ abweichen.

Auch hier kann die Abweichung der einzelnen Lichtstrahlen über den angegebenen Bereich streuen. In jedem Fall sollte die Leuchtdichte dieses Lichtbündels ein Vielfaches, wenigstens das Fünffache der Leuchtdichte des parallel zur Behälterachse verlaufenden Lichtbündels sein.

Mit dem Merkmal "Neigung der Lichtstrahlen gegenüber der Behälterachse" wird ein Zustand bezeichnet, bei dem die einzelnen Lichtstrahlen nicht parallel zur Behälterachse, sondern geneigt gegenüber der Behälterachse verlaufen. Hierbei kommt sowohl ein Verlauf der Lichtstrahlen in Betracht, bei dem diese die Behälterachse schneiden, als auch ein windschiefer Verlauf. Besonders günstig ist es, wenn dabei die Leuchtdichte der einzelnen Lichtbündel in Umfangsrichtung zur Behälterachse gleichmäßig verteilt ist, d. h., wenn die Lichtstrahlen auf den Mantelflächen von Kegeln verlaufen, deren Achse mit der Behälterachse übereinstimmt.

Die Beleuchtung des Behälterbodens mit Lichtbündeln unterschiedlicher Neigung gegenüber der Behälterachse wird mittels einer Anordnung durchgeführt, bei der unter dem Boden des transparenten Behälters eine Beleuchtungseinrichtung angeordnet ist und bei der in Weiterbildung der Erfindung die Beleuchtungseinrichtung von einem nach oben offenen, gleichachsig zum Behälter angeordneten zylindrischen Rohrkörper umgeben ist, dessen Innendurchmesser größer als die Querschnittsabmessung des Behälters und dessen zylindrische Innenwand mit einem die Lichtstrahlen diffus streuenden Belag versehen ist, und bei der der Beleuchtungseinrichtung Flächen und/oder Blenden am Boden und/oder in einer Zwischenebene des Rohrkörpers und/oder konzentrisch zur Achse des Rohrkörpers zugeordnet sind, die eine spiegelnde und/oder diffus streuende Oberfläche aufweisen.

Durch die Verwendung diffus streuender Beläge und gegebenenfalls spiegelnder Flächen wird dabei die erwünschte Leuchtdichte und die erwünschte Neigung der Lichtstrahlen erreicht. Die diffus streuenden Beläge der einzelnen Flächen innerhalb des Rohrkörpers können dabei unterschiedliche Reflexionsfaktoren aufweisen.

Eine einfache und zugleich wirksame Beleuchtungsanordnung ist dadurch gegeben, daß die Beleuchtungseinrichtung unmittelbar über dem mit einem diffus streuenden Belag versehenen Boden des Rohrkörpers in dessen Achse angeordnet ist und daß über der Beleuchtungseinrichtung eine den Boden des Behälters gegen direkte Lichteinstrahlung abschirmende Blende angeordnet ist. In diesem Fall führen die am Behälterboden und an der Blende diffus reflektierten Lichtstrahlen zur achsparallelen oder annähernd achsparallelen Beleuchtung des Behälterbodens, während die an der zylindrischen Innenwand des Rohrkörpers reflektierten Lichtstrahlen - je nach Dimensionierung und Anordnung der abschirmenden Blende - auf den Behälterboden unter einem Winkel auftreffen, der gegenüber der Behälterachse um ca. 5 bis 45 $^{\circ}$ geneigt ist. Unterschiedliche Leuchtdichten der einzelnen Lichtbündel werden dadurch erreicht, daß die diffus streuenden Beläge an der Innenwand und auf dem Boden des Rohrkörpers unterschiedliche Reflexionsfaktoren aufweisen.

d) Ausführungsbeispiele

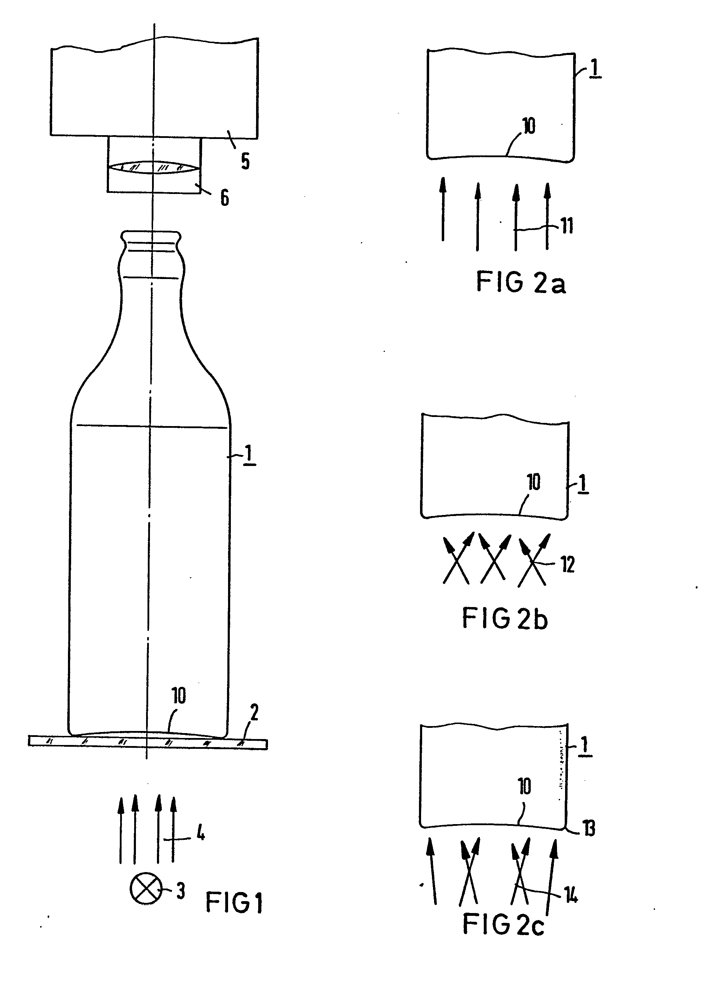

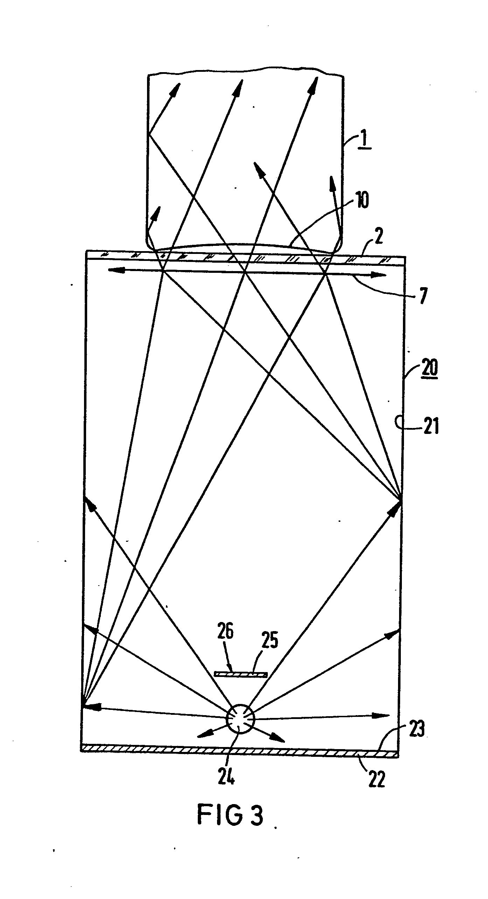

Ausführungsbeispiele von Anordnungen zur Durchführung des neuen Verfahrens sind in den Figuren 3 bis 6 dargestellt, während das neue Verfahren anhand der Darstellung in den Figuren 1 und 2 zunächst allgemein erläutert wird.

Gemäß Figur 1 ist ein transparenter Behälter 1 in Form einer Flasche auf einer durchscheinenden Platte 2 angeordnet. Der Flaschenboden 10 wird von unten mit dem von einer blitzartigen Beleuchtungseinrichtung 3 ausgehenden Lichtbündel 4 beleuchtet, deren Aufnahmeröhre Speichereigenschaft hat. Über der Flasche 1 ist die Fernsehkamera 5 mit dem Objektiv 6 angeordnet. Die optische Achse der Fernsehkamera 5 ist gleichachsig zur Längsachse der Flasche 1 angeordnet.

Gemäß Figur 2a erfolgt die allgemeine Aufhellung des Flaschenbodens mit Hilfe des Lichtbündels 11, dessen Lichtstrahlen parallel oder in einem Winkel von bis zu 5 $^{\circ}$ zur Flaschenachse verlaufen.

Gemäß Figur 2b ist ein weiteres, zur Behälterachse rotationssymmetrisches Lichtbündel 12 vorgesehen, dessen Lichtstrahlen auf den Flaschenboden 10 unter einem Winkel von 10 bis 45 $^{\circ}$ auftreffen. Mit Hilfe dieses Lichtbündels erfolgt eine Aufhellung von Prägungen und Riffelungen im Flaschenboden.

Gemäß Figur 2c ist ein drittes Lichtbündel 14 vorgesehen, dessen Lichtstrahlen ebenfalls rotationssymmetrisch zur Behälterachse verlaufen und auf den Flaschenboden 10 unter einem Winkel auftreffen, der gegenüber der Flaschenachse um 5 bis 15 $^{\circ}$ geneigt ist. Mit Hilfe dieses Lichtbündels erfolgt eine Aufhellung des Übergangsbereiches 13 vom Flaschenboden zur Flaschenwand.

Die erwünschte unterschiedliche Neigung der Lichtstrahlen gegenüber der Behälterachse kann gemäß Figur 3 mit einer Anordnung erreicht werden, bei der unter dem Boden 10 der Flasche 1 eine Beleuchtungseinrichtung angeordnet ist, die im wesentlichen aus dem Rohrkörper 20 und der Blitzlampe 24 besteht. Der zylindrische Rohrkörper 20 ist nach oben hin mit der durchsichtigen Glasplatte 2 abgeschlossen, während im Bodenbereich der kreisförmige Deckel 22 angeordnet ist, der auf seiner Innenseite einen diffus reflektierenden Belag 23 trägt. Über der in der Achse des Rohrkörpers 20 angeordneten Blitzlampe 24 befindet sich die kreisförmige Blende 25, die auf der Oberseite und gegebenenfalls auch auf der Unterseite mit einem diffus streuenden Belag versehen ist. Ein diffus streuender Belag 21 befindet sich auch auf der inneren Oberfläche des Rohrkörpers 20. Weiterhin ist unmittelbar unterhalb der Glasplatte 2 eine optische Linse 7 in Form einer Fresnel-Linse angeordnet.

Durch die kreisförmige Blende 25 wird eine direkte Beleuchtung des Flaschenbodens 10 durch die Blitzlampe 24 unterbunden, während die Fresnel-Linse 7 vor allem im Übergangsbereich Flaschenboden/Flaschenwand den Lichtstrahlen die erforderliche Neigung von 5 bis 15 ° erteilt.

Die in Figur 3 dargestellte Anordnung dient also dazu, Lichtstrahlen unterschiedlicher Intensität in einem Winkelbereich von 0 bis 45 ° gegenüber der Behälterachse zu erzeugen. Dabei ergeben sich die schräg in den Flaschenboden 10 einfallenden Lichtstrahlen durch Reflexion der von der Blitzlampe 24 ausgehenden Lichtstrahlen an der Innenwand des Rohrkörpers 20. Diese ist mit einem diffus streuenden Belag, beispielsweise einem mattweißen Lack, versehen. Die direkt den Flaschenboden 10 ausleuchtenden Lichtstrahlen stammen von der ebenfalls diffus streuenden Bodenfläche 22 und der Oberfläche der Blende 25. Die Reflexion dieser Flächen muß niedriger gewählt werden als die Reflexion der Innenwand des Rohrkörpers 20, damit die Leuchtdichte der schräg einfallenden Lichtstrahlen größer ist als die Leuchtdichte der parallel zur Behälterachse in den Flaschenboden einfallenden Lichtstrahlen.

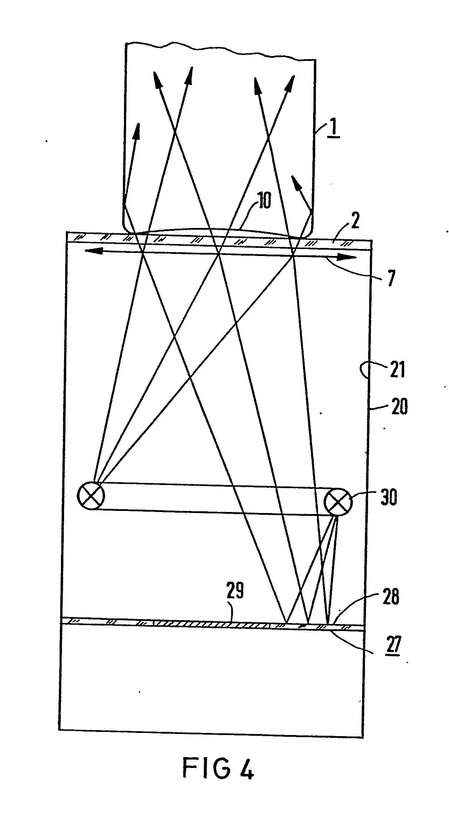

Lichtstrahlen unterschiedlicher Neigung und Intensität können auch mit der in Figur 4 dargestellten Anordnung erzeugt werden. Danach befindet sich in dem Rohrkörper 20, der auf der Innenseite mit dem diffus streuenden Belag 21 versehen ist, konzentrisch zur Achse des Rohrkörpers und damit konzentrisch zur Achse der Glasflasche 1 eine Ringblitzlampe 30, deren Durchmesser größer als der Durchmesser der Glasflasche 1 ist. Die Ringblitzlampe 30 befindet sich über einem Zwischenboden 27, der im äußeren Bereich verspiegelt ist und im inneren Bereich mit einem diffus reflektierenden Belag versehen ist. Die Reflexion der von der Ringblitzlampe 30 ausgehenden Lichtstrahlen an der kreisförmigen Fläche 29 führt zur achsparallelen Beleuchtung des Flaschenbodens 10, während die von der Ringblitzlampe 30 direkt ausgehenden und an der kreisringförmigen Spiegelfläche 28 reflektierten Lichtstrahlen auf den Flaschenboden 10 jeweils unter dem gewünschten Winkel auftreffen.

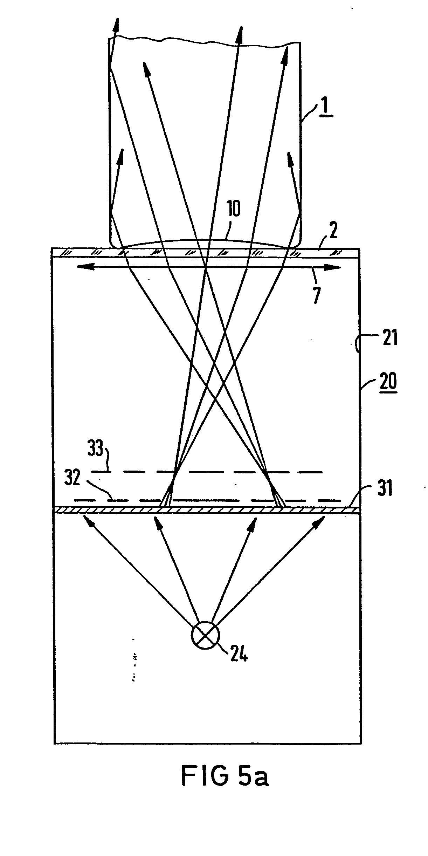

Bei der Anordnung gemäß Figur 5a ist im zylindrischen Rohrkörper 20 ein Zwischenboden 31 aus Mattglas angeordnet, unter dem sich die Blitzlampe 24 in der Achse des Rohrkörpers befindet. Über dem Zwischenboden 31 sind in zwei Ebenen die kreisförmigen bzw. kreisringförmigen Blenden 32 und 33, sogenannte Lichtstoppstreifen, angeordnet, deren Öffnungen radial gegeneinander versetzt sind. Dadurch wird erreicht, daß von der Mattglasscheibe 31 direkt ausgehende Lichtstrahlen nur in dem gewünschten Winkelbereich von 5 bis 45 $^{o}$ auf den Flaschenboden der Glasflasche 1 auftreffen. Im übrigen sind die Blenden 32 und 33 mit einem diffus reflektierenden Belag versehen, so daß die im Bereich zwischen der Mattglasscheibe 31 und der Glasplatte 2 auf die Oberflächen der Blenden 32 und 33 reflektierten Lichtstrahlen zu der gewünschten achsparallelen Beleuchtung der Glasflasche 1 führen. Es kommen aber auch Blenden aus transparentem, lichtdämpfendem Material in Betracht.

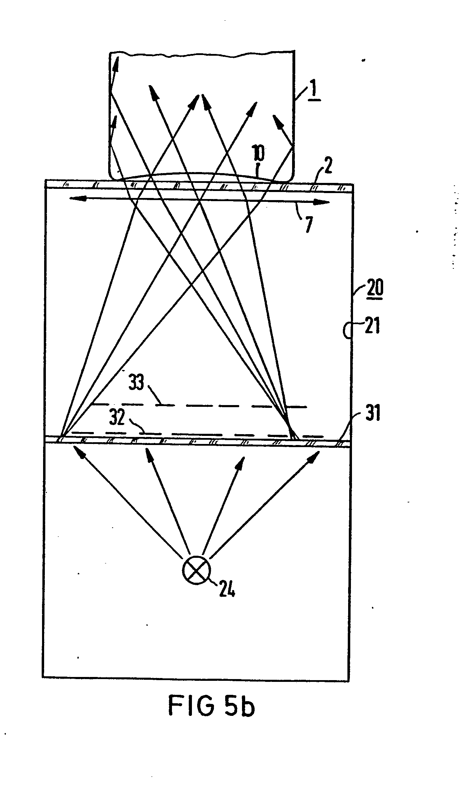

Während in Figur 5a diejenigen Lichtstrahlen dargestellt sind, die zu einer Aufhellung des Übergangsbereiches Flaschenboden/Flaschenwand führen, sind in Figur 5b diejenigen Lichtstrahlen dargestellt, die zu einer Aufhellung von Bodenprägungen führen.

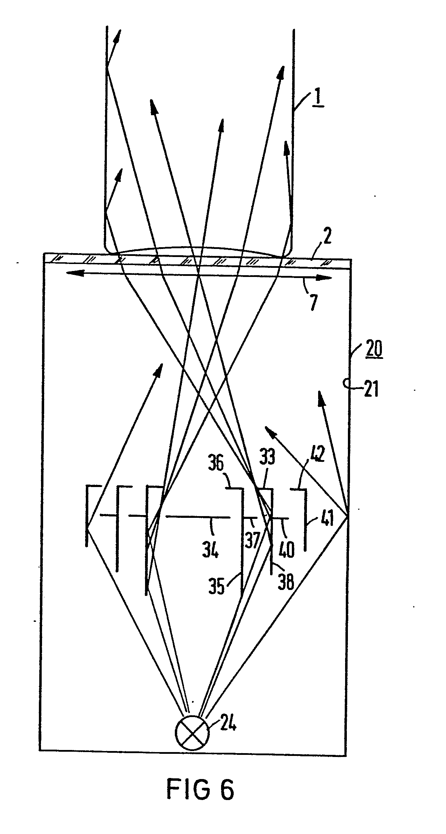

Bei der Anordnung gemäß Figur 6 ist die Blitzlampe 24 im Bodenbereich des mit einem diffus streuenden Belag 21 versehenen Rohrkörpers 20 angeordnet. Zwischen der Blitzlampe 24 und der Glasplatte 2 sowie der vorgeschalteten Fresnel-Linse 7 befinden sich konzentrisch zur Achse des Rohrkörpers mehrere Reflektorblenden, die aus der Kreisblende 34 und den zylindrischen Reflektoren 35, 38 und 41 mit den zugeordneten Kreisringblenden 36 und 37 bzw. 39 und 40 sowie 42 bestehen. Die von der Blitzlampe 24 ausgehenden Lichtstrahlen werden an der inneren Oberfläche der Reflektoren 35, 38 und 41 reflektiert und infolge der Abschattung mit Hilfe der Kreisringblenden 36, 37, 39, 40 und 42 unter den gewünschten Winkeln auf den Flaschenboden 10 gelenkt. Die kreisringförmigen Blenden sowie die Kreisblende 34 sind mit einem diffus reflektierenden Belag versehen, um die Ausleuchtung des Flaschenbodens mit parallel zur Achse des Rohrkörpers 20 verlaufenden Lichtstrahlen zu gewährleisten. Die Kreisblende 34 verhindert im übrigen eine direkte Beleuchtung des Flaschenbodens.

6 Figuren
12 Ansprüche

- 10 -   VPA 80 P 3534

<u>Patentansprüche</u>

1. Verfahren zur Ermittlung von Verunreinigungen im Bodenbereich von transparenten Behältern, insbesondere von Glasflaschen, bei dem der Behälterboden von unten beleuchtet sowie durch die Öffnung des Behälters mittels einer Fernsehkamera beobachtet und das Videosignal der Fernsehkamera elektronisch ausgewertet wird und bei dem die Lichtstrahlen des zur Beleuchtung dienenden Lichtbündels parallel oder annähernd parallel zur senkrechten Achse des Behälters verlaufen, d a d u r c h   g e k e n n - z e i c h n e t ,   daß der Behälterboden (10) mit Hilfe wenigstens eines weiteren Lichtbündels (12,14) beleuchtet wird, dessen Lichtstrahlen von der senkrechten Achse des Behälters rotationssymmetrisch um wenigstens 5 $^{\circ}$ abweichen und dessen Leuchtdichte um ein Vielfaches größer als die Leuchtdichte des erstgenannten Lichtbündels (11) ist.

2. Verfahren nach Anspruch 1, d a d u r c h   g e - k e n n z e i c h n e t ,   daß die Lichtstrahlen des zweiten Lichtbündels (12) von der senkrechten Achse des Behälters um ca. 10 bis 45 $^{\circ}$ abweichen.

3. Verfahren nach Anspruch 2, d a d u r c h   g e - k e n n z e i c h n e t ,   daß die Leuchtdichte des zweiten Lichtbündels (12) ein Vielfaches der Leuchtdichte des ersten Lichtbündels (11) ist.

4. Verfahren nach einem der Ansprüche 1 bis 3, d a - d u r c h   g e k e n n z e i c h n e t ,   daß der Behälterboden mit einem zweiten bzw. dritten Lichtbündel (14) beleuchtet wird, dessen Lichtstrahlen von der senkrechten Achse des Behälters um ca. 5 bis 15 $^{\circ}$ abweichen.

5. Verfahren nach Anspruch 4, d a d u r c h  g e -
k e n n z e i c h n e t ,  daß die Leuchtdichte des
zweiten bzw. dritten Lichtbündels (14) ein Vielfaches der
Leuchtdichte des ersten Lichtbündels (11) ist.

6. Anordnung zur Durchführung des Verfahrens nach Anspruch 1, bei der unter dem Boden des transparenten Behälters eine Beleuchtungseinrichtung angeordnet ist,
d a d u r c h  g e k e n n z e i c h n e t ,  daß die
Beleuchtungseinrichtung (24,30) von einem nach oben offenen, gleichachsig zum Behälter (1) angeordneten zylindrischen Rohrkörper (20) umgeben ist, dessen Innendurchmesser größer als die Querschnittsabmessung des Behälters
und dessen zylindrische Innenwand mit einem die Lichtstrahlen diffus streuenden Belag (21) versehen ist,
und daß der Beleuchtungseinrichtung (24,30) Flächen (22,
27) und/oder Blenden (34 - 41,25) am Boden und/oder in
einer Zwischenebene des Rohrkörpers und/oder konzentrisch
zur Achse des Rohrkörpers zugeordnet sind, die eine spiegelnde und/oder diffus streuende Oberfläche aufweisen.

7. Anordnung nach Anspruch 6, d a d u r c h  g e -
k e n n z e i c h n e t ,  daß der Rohrkörper (20) nach
oben mit einer Glasplatte (2) abgedeckt ist.

8. Anordnung nach Anspruch 7, d a d u r c h  g e -
k e n n z e i c h n e t ,  daß unmittelbar unterhalb der
Glasplatte eine Linse, insbesondere eine Fresnel-Linse
(7) angeordnet ist.

9. Anordnung nach einem der Ansprüche 6 bis 8, d a -
d u r c h  g e k e n n z e i c h n e t ,  daß die Beleuchtungseinrichtung (24) unmittelbar über dem mit einem
diffus streuenden Belag (23) versehenen Boden (22) des
Rohrkörpers in dessen Achse angeordnet ist und daß über der Beleuchtungseinrichtung (24) eine den Boden (10) des Behälters (1) gegen direkte Lichteinstrahlung abschirmende Blende (25) angeordnet ist.

10. Anordnung nach einem der Ansprüche 6 bis 8, d a - d u r c h  g e k e n n z e i c h n e t, daß die Beleuchtungseinrichtung als konzentrisch zur Achse des Rohrkörpers angeordnete Ringlampe (30) ausgebildet ist, deren Durchmesser größer als die Querschnittsabmessung des Behälters (1) ist, und daß unterhalb der Ringlampe ein spiegelnder Zwischenboden (27) angeordnet ist, dessen innere Kreisfläche mit einem diffus streuenden Belag (29) versehen ist.

11. Anordnung nach einem der Ansprüche 6 bis 8, d a - d u r c h  g e k e n n z e i c h n e t, daß die Beleuchtungseinrichtung (24) in der Achse des Rohrkörpers (20) angeordnet ist und daß sich über der Beleuchtungseinrichtung ein Zwischenboden (31) aus Mattglas befindet, dem auf der Oberseite ringförmige Lichtstoppstreifen (32, 33) zugeordnet sind, die entweder aus transparentem, lichtdämpfendem Material bestehen oder mit einem diffus streuenden Belag versehen sind.

12. Anordnung nach einem der Ansprüche 6 bis 8, d a - d u r c h  g e k e n n z e i c h n e t, daß die Beleuchtungseinrichtung (24) in der Achse des Rohrkörpers angeordnet ist und daß zwischen der Beleuchtungseinrichtung und dem Behälter (1) konzentrisch zueinander sowie zur Achse des Rohrkörpers (20) rohrartige Reflektorblenden (34 - 41) angeordnet sind, deren Blenden (34,36,37, 39,40,42) mit einem diffus streuenden Belag versehen sind.

FIG 1

FIG 2a

FIG 2b

FIG 2c

FIG 3

FIG 4

FIG 5a

FIG 5b

0045407

FIG 6

Europäisches
Patentamt

**EUROPÄISCHER RECHERCHENBERICHT**

**0045407**

Nummer der Anmeldung

EP 81 10 5512

| Kategorie | **EINSCHLÄGIGE DOKUMENTE** | | KLASSIFIKATION DER ANMELDUNG (Int Cl³) |
|---|---|---|---|
| | Kennzeichnung des Dokuments mit Angabe, soweit erforderlich, der maßgeblichen Teile | betrifft Anspruch | |
| AD | DE - A - 2 848 316 (SIEMENS)  <br> * Seiten 14,15; Figur 1 * | 1 | G 01 N 21/90 |
| A | PATENT ABSTRACTS OF JAPAN, Band 3, Nr. 94(E-129), 10. August 1979, Seite 75E129 <br> & JP - A - 54 72094 (HITACHI DENSHI K.K.) <br> * Insgesamt * | 1,2 | |
| A | US - A - 3 651 937 (H. KRONSEDER) <br> * Spalte 5; Figur 8 * | 1,2 | RECHERCHIERTE SACHGEBIETE (Int. Cl.³) <br><br> G 01 N 21/90 |
| A | US - A - 3 478 221 (J. SAINSBURY) <br> * Spalte 3; Figur 1 * | 1 | |
| A | US - A - 3 434 594 (R. HUSOME) <br> * Spalte 12; Figur 8 * | 1,2 | |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 09-11-1981 | BOEHM |

EPA form 1503.1 06.78